# EUROPÄISCHE PATENTSCHRIFT

(11) **EP 2 389 128 B1**
(45) Veröffentlichungstag und Bekanntmachung des Hinweises auf die Patenterteilung: **26.03.2014**
(21) Anmeldenummer: 10701647.9
(22) Anmeldetag: 14.01.2010
(51) Int. Cl.: A61B 17/70

(54) **BANDSCHEIBENENTLASTUNGSSTÜTZE**
INTERVERTEBRAL DISK STRAIN-RELIEF SUPPORT
SUPPORT POUR SOULAGER LES DISQUES INTERVERTÉBRAUX

(30) Priorität: 21.01.2009 CH 912009
(43) Veröffentlichungstag der Anmeldung: 30.11.2011
(73) Patentinhaber: TICOM GmbH, 90765 Fürth (DE)
(72) Erfinder: HOLZWARTH, Ulrich, 90542 Eckental (DE)
(74) Vertreter: Rau, Schneck & Hübner Patentanwälte Rechtsanwälte PartGmbB
(86) Internationale Anmeldenummer: PCT/EP2010/050420
(87) Internationale Veröffentlichungsnummer: WO 2010/084079

(56) Entgegenhaltungen:
- WO-A1-2005/009300
- WO-A1-2006/064356
- US-A1- 2008 208 344

## Beschreibung

### Technisches Gebiet

Die Erfindung betrifft eine Bandscheibenentlastungsstütze als Implantat zwischen Dornfortsätzen benachbarter Wirbelkörper, umfassend ein Trägerelement mit einer Aussparung und mit ersten Rückhalteflügeln, sowie ein in die Aussparung des Trägerelements einschiebbares Zentralelement mit zweiten Rückhalteflügeln, wobei im implantierten Zustand die Rückhalteflügel aussen an den Dornfortsätzen benachbarten Wirbelkörper anliegen und sich die Dornfortsätze auf gegenüberliegenden Druckflächen auf dem Trägerelement und/oder auf dem Zentralelement abstützen.

### Stand der Technik

Aus der WO 2006/064356 ist ein Implantat zur Behandlung der lumbalen Spinalkanalstenose mit einem Abstandhalter benachbarter Dornfortsätze bekannt. Es handelt sich dabei um ein im wesentlichen aus zwei Komponenten bestehendes Implantat. Die erste Komponente wird seitlich zwischen zwei Dornfortsätze eingeschoben und anschliessend wird die zweite Komponente in eine dafür vorgesehene Aussparung der ersten Komponente eingeschoben. Die erste Komponente verfügt über zwei eingeklappte Flügel, welche beim Einschieben der zweiten Komponente automatisch ausgeklappt werden. Nach dem Zusammenfügen der Komponenten werden diese miteinander fixiert, beispielsweise verschraubt. Zusammen mit zwei weiteren Flügeln, die fest an der zweiten Komponente angebracht sind, ergibt sich ein System, das sich zwischen zwei Dornfortsätzen fest hält. Die erste und/oder die zweite Komponente bildet einen transversal verlaufende Wälzkörper des Implantats zur Entlastung der Bandscheibe, indem er einen Abstandhalter zwischen benachbarten Dornfortsätzen bildet.

Solche Implantate haben sich in der Praxis bewährt. Nachteilig ist einzig, dass der Wälzkörper absolut starr ist.

Verschiedene Hersteller haben versucht, flexible Materialien, insbesondere Kunststoffe als Materialien für den Wälzkörper zu verwenden. Da diese Materialien aber bei den hier auftretenden Kräften ungenügend sind und bezüglich dem Langzeitverhalten wenig erprobt, stellen sie ein gewisses Risiko dar.

Die US 2008/0208344 A1 offenbart eine Vielzahl von Bandscheibenentlastungsstützen. Die Bandscheibenentlastungsstütze gemäß Figuren 66, 67 umfasst ein Oberteil und ein Unterteil. Das Oberteil und das Unterteil sind schwenkbar miteinander verbunden und weisen jeweils einander gegenüberliegende Druckflächen auf. Benachbart zu den Druckflächen verlaufen Federschlitze. Zwischen den Federschlitzen und den zugehörigen Druckflächen liegen federnde Brücken vor. Nachteilig an dieser bekannten Bandscheibenentlastungsstütze ist, dass die Federwirkung der Brücken nicht zufriedenstellend ist.

Aus der WO 2005/009300 A1 ist eine weitere Bandscheibenentlastungsstütze bekannt. Diese Bandscheibenentlastungsstütze hat Fluidkammern zur Erhöhung der Dämpfungseigenschaften.

### Darstellung der Erfindung

Aufgabe der vorliegenden Erfindung ist es, ein Implantat eingangs erwähnter Art anzugeben, das eine gewisse Flexibilität zulässt, bei Verwendung erprobter medizinaltechnisch zugelassener Metalllegierungen unter Meidung von Kunststoffen.

Die Aufgabe wird gelöst durch die Kennzeichen des unabhängigen Patentanspruchs.

Die der Erfindung zugrunde liegende Idee besteht darin, dass mindestens ein frei liegender Federschlitz, welcher sich transversal zur sagitalen Verlaufsrichtung der Wirbelsäule erstreckt, zwischen zwei gegenüberliegenden Druckflächen in mindestens einem der Elemente derart angeordnet ist, dass dadurch zwischen dem Federschlitz und einer der Druckflächen des Implantates eine federnde Brücke ausgestaltet wird, welche bei Belastung der Dornfortsätze eine federnde Wirkung hat. Durch mindestens zwei Federschlitze sind zwei Brücken ausgestaltet, die je einem Dornfortsatz zugeordnet sind. Alle Federschlitze verlaufen parallel zueinander.

Weitere vorteilhafte erfindungsgemässe Ausgestaltungen sind in den abhängigen Ansprüchen angegeben.

### Kurze Beschreibung der Zeichnungen

Im Folgenden wird die Erfindung unter Beizug der Zeichnungen näher erklärt. Es zeigen
- Fig. 1: ein zwischen zwei benachbarten Dornfortsätzen eines Wirbelkörpers angeordnetes Implantat in seiner Einbaulage, von der Seite her betrachtet;
- Fig. 2: eine Darstellung eines erfindungsgemässen Implantats in zusammengesetztem Zustand in vier verschiedenen perspektivischen Ansichten a, b, c, d;
- Fig. 3: eine schematische Darstellung von Trägerelement und Zentralelement, in zwei verschiedenen Ansichten von oben her betrachtet, (a) halb auseinander und (b) von der Seite betrachtet.

### Wege zur Ausführung der Erfindung

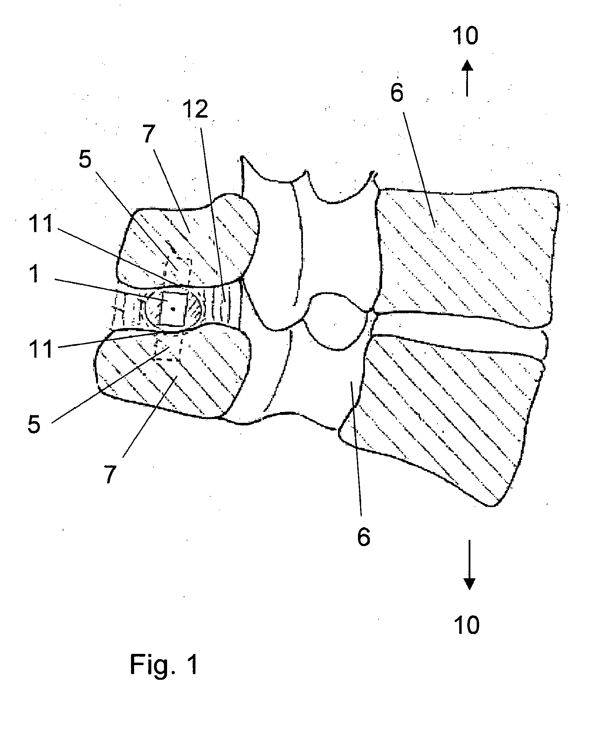

In Fig. 1 ist ein Teil der Wirbelsäule 10 eines Patienten dargestellt mit zwei benachbarten Wirbelkörpern 6 und deren Dornfortsätze 7. Die Wirbelsäule 10 erstreckt sich in dieser Darstellung in Richtung der Pfeile. Zwischen zwei benachbarten Dornfortsätzen 7 verläuft das interspinale Ligament 12. Dieses wird von einer Bandscheibenentlastungsstütze 1 durchquert und beidseitig beider benachbarter Dornfortsätze 7 von zwei Rückhalteflügel Paaren 5, 5' gegen transversale Verschiebungen in seiner Position gehalten. Die Bandscheibenentlastungsstütze 1 ist mittig für die Auflage der Dornfortsätze 7 vorzugsweise tailliert ausgestaltet und somit einer Selbstzentrierung unterworfen, sodass sie zusätzlich gegen Verschiebungen gesichert ist.

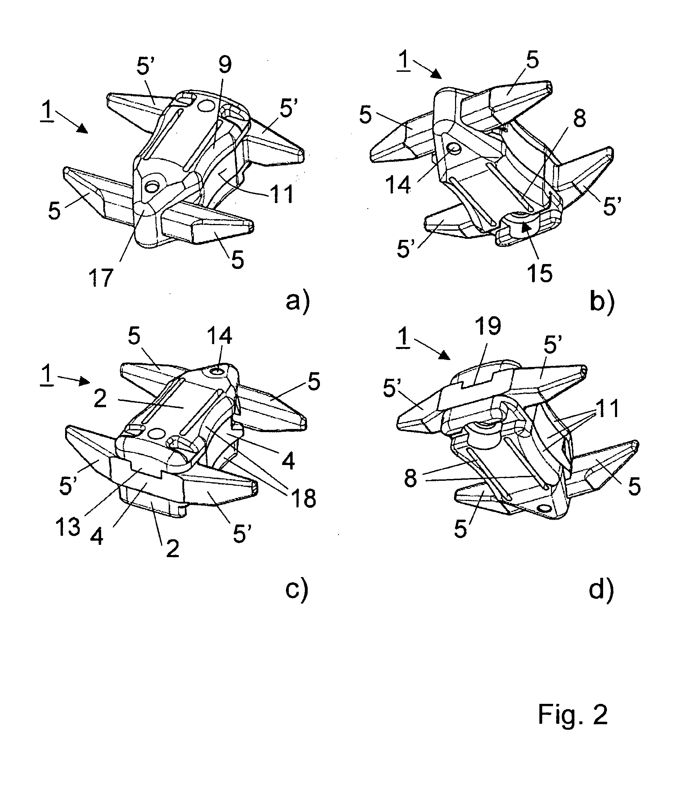

Fig. 2 zeigt die erfindungsgemässe Bandscheibenentlastungsstütze 1 in vier verschiedenen Perspektiven und in einem fertig montiertem Zustand. Die vier Rückhalteflügel 5, 5' liegen im eingesetzten Zustand jeweils an hier nicht dargestellten Dornfortsätzen 7 an.

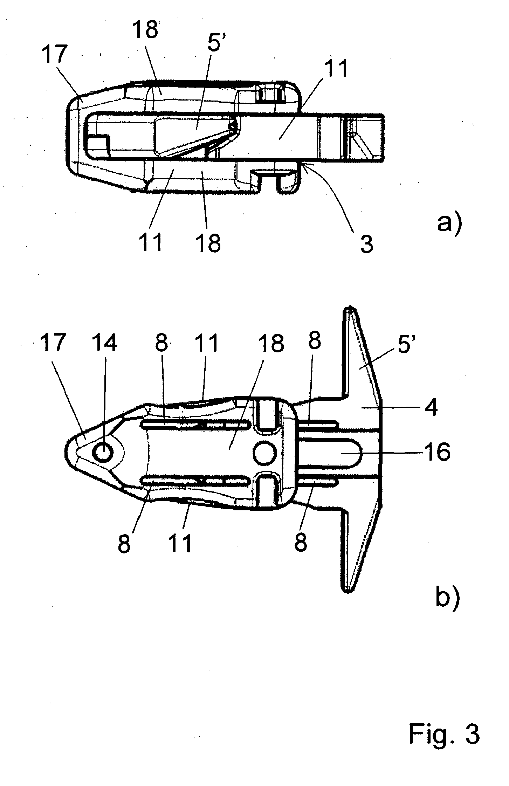

Fig. 3 a) und b) zeigen schematische Darstellungen der Bandscheibenentlastungsstütze 1 von oben her betrachtet (a) und von der Seite betrachtet (b), jeweils in teilweise auseinandergezogenen Zuständen. Die beweglichen Rückhalteflügel 5 am Zentralelement 4, die sich um die Drehachse 14 bewegen können, liegen hier noch zwischen den Stützwänden 18 des Trägerelementes 2.

Die Bandscheibenentlastungsstütze 1 besteht im Wesentlichen aus zwei Komponenten, namentlich aus einem Trägerelement 2 mit einer Aussparung 3 und aus einem Zentralelement 4, das in die Aussparung 3 des Trägerelements 2 passig eingefügt werden kann.

Das Trägerelement 2 ist durch die Aussparung 3 in zwei parallele Stützwände 18 gegliedert, die im Bereich eines Einführungskopfes 17 einstückig miteinander verbunden sind. Im Bereich des Einführungskopfes 17 ist eine Drehachse vorhanden, um welche die beweglichen Flügel 5 schwenken können. In einer ersten Phase des Einbaus sind die Flügel zwischen den beiden Stützwänden 18 liegend. Dies erlaubt den seitlichen Einschub des Trägerelements 2 zwischen zwei benachbarten Wirbelkörperfortsätzen. Hierdurch lässt sich von derselben Seite her das Zentralelement 4 mit seinen starren Flügeln 5' in die Aussparung 3 einschieben. Dies wird erleichtert durch eine auf der Innenseite einer der beiden Stützwände 18 angebrachten Schiene 13, die sich vom offenen Ende des Trägerelementes 2 bis zu dessen Einführungskopf 17 erstreckt, sowie durch eine gegengleiche Nut 19 im Zentralelement 4.

Beim Einschieben des Zentralelements 4 in die Aussparung 3 des Trägerelements 2 werden die eingeklappten Rückhalteflügel 5 schliesslich automatisch in die Endposition ausgefahren. Eine Schraube 15 fixiert schliesslich die beiden Elemente 2, 4 und hält deren relative Position zueinander fest. Vorzugsweise verfügt das Zentralelement 4 über ein Langloch 16, damit die relative Position zwischen Zentralelement 4 und Trägerelement den Breiten der Dornfortsätze des Patienten angepasst werden können.

Im implantierten Zustand stützen sich die Dornfortsätze 7 auf gegenüberliegenden Druckflächen 11 auf dem Trägerelement 2 und/oder auf dem Zentralelement 4 ab. Hier beschriebene Bandscheibenentlastungsstützen sind bekannt aus der WO 2006/064356.

Die in den Figuren dargestellten erfindungsgemässen Bandscheibenentlastungsstützen 1 weisen zusätzlich zu den Bandscheibenentlastungsstützen 1 nach dem Stand der Technik mindestens einen frei liegenden Federschlitz 8 auf, welcher sich transversal zur sagitalen Verlaufsrichtung der Wirbelsäule 10 erstreckt. Dieser Federschlitz 8 befindet sich parallel zu und zwischen den beiden gegenüberliegenden Druckflächen 11 in mindestens einem der Elemente 2, 4. Der Federschlitz 8 ist derart angeordnet, dass zwischen ihm und der nächsten Druckfläche 11 eine Federnde Brücke 9 ausgestaltet wird. Bei einer Belastung der Dornfortsätze 7 gibt diese Brücke 9 federnd nach, wodurch die gewünschte Flexibilität erreicht ist. Der Federschlitz 8 ist vorzugsweise nahe einer belastbaren Druckfläche 11 angeordnet, damit die gewünschte Federwirkung entsteht.

Nach dem Stand der Technik ist ein mittlerer Schlitz im Zentralelement 4 als Langloch 16 ausgestaltet. Dieses gewährleistet, dass eine Schraube 15 bei der Verbindung der beiden Elemente 2, 4 einen einstellbaren Abstand der Rückhalteflügelpaare 5, 5' gewährleisten kann, entsprechend den Breiten der Dornfortsätze 7 des Patienten. Dieses Langloch 16 erfüllt jedoch nicht die Funktion eines Federschlitzes 8, unterstützt jedoch gegebenenfalls dessen Wirkung.

Der erfindungsgemäße Federschlitz 8 ist nahe bei einer Druckfläche 11 angebracht, damit eine dünne, federnde Brücke 9 ausgestaltet werden kann. Je dünner die Brücke 9, d. h. je geringer die Distanz zwischen einer Druckfläche 11 und den ihr zugeordneten Federschlitzen 8 ist, desto nachgiebiger ist die Bandscheibenentlastungsstütze 1.

Es sind durch mindestens zwei Federschlitze 8 zwei gegenüberliegende Brücken 9 ausgestaltet, die je einem Dornfortsatz 7 zugeordnet sind. Solche Federschlitze 8 sind an dem Element oder an den Elementen 2, 4 anzubringen, an welchen die Dornfortsätze 7 an Druckflächen 11 aufliegen. Wenn der Druck auf beide Seitenwände 18 verteilt ist, sollen insgesamt vier Federschlitze 8 angebracht sein, zwei auf jeder Seitenwand 18, je einem Dornfortsatz 7 zugeordnet. Verteilt sich der Druck zusätzlich noch auf das Zentralelement, so sind insgesamt sechs Federschlitze 8 angebracht, die vier beschriebenen an den Seitenwänden 18 sowie zwei am Zentralelement, bei jedem Dornfortsatz einer.

Vorzugsweise sollten Federschlitze 8 nahe allen Auflageflächen 11 angebracht werden, damit eine optimale Federung erreicht wird. Je nach Ausgestaltung der Höhen von Trägerelement 2 und Zentralelement 4 sind unterschiedlich viele Federschlitze 8 anzubringen, insbesondere zwei, vier oder sechs.

Ist die Höhe der Elemente 2, 4 ungleich ausgestaltet, so dass Druckflächen 11 für die Dornfortsätze 7 nur an einem der Elemente 2, 4 oder einem Teil des Trägerelements 2 vorgesehen sind, so genügen zwei Federschlitze 8 beidseits an diesem Element 2 resp. 4. Sind aber das Trägerelement 2 und das Zentralelement 4 gleich hoch, sodass jeder Dornfortsatz 7 drei Auflageflächen 11 aufweist, so sollten insgesamt sechs Federschlitze 8 an der Bandscheibenentlastungsstütze 1 angebracht sein, nämlich beidseitig der Aussparung 3 des Trägerelements 2 sowie am Zentralelement 4, jeweils je eines im Bereich jedes Dornfortsatzes 7.

Jede Druckfläche 11 jedes Elementes 2, 4 soll durch einen Federschlitz 8 jeweils zu einer federnden Brücke 9 ausgestaltet sein, sodass im implantierten Zustand beide Dornfortsätze 7 vollständig gefedert auf den Druckflächen 11 aufliegen. Vorzugsweise sollten die Federschlitze 8 im Wesentlichen den ganzen Bereich der Elemente 2, 4 zwischen den beiden Paaren von Rückhalteflügeln 5, 5' umfassen, damit die Federwirkung der Brücke 9 bei Belastung auch gewährleistet ist.

Prinzipiell müssen die Federschlitze 8 beidseitig an den Stützwänden 18 gemäß einer nicht-erfindungsgemäßen Ausführungsform weder parallel noch in gleichen Abständen von den Druckflächen 11 entfernt verlaufen. Dies kann aber dazu führen, dass die Federwirkung der Brücken 9 unterschiedlich ist, so dass Scherbewegungen auftreten können. Dies ist unerwünscht und daher werden die Federschlitze 8 sowohl in den beiden Stützwänden als auch im Trägerelement in der Größe und Verlaufsrichtung gleich und parallel verlaufend angeordnet, sodass im zusammengebauten Zustand alle Federschlitze 8, die einem Dornfortsatz 7 nahe liegen, deckungsgleich übereinander liegen.

Obwohl es genügen würde, nur einem Dornfortsatz zugeordnet in beiden Stützwänden und dem Trägerelement 4 je ein Federschlitz 8 anzuordnen, wird bevorzugt die in den Zeichnungen dargelegte Version mit beidseitiger Anordnung der Federschlitze 8 realisiert.

### Bezugszeichenliste

- 1: Bandscheibenentlastungsstütze
- 2: Trägerelement
- 3: Aussparung
- 4: Zentralelement
- 5: 5' Rückhalteflügel
- 6: Wirbelkörper
- 7: Dornfortsatz
- 8: Federschlitz
- 9: federnde Brücke
- 10: Wirbelsäule
- 11: Druckfläche
- 12: Ligament
- 13: Schiene
- 14: Drehachse
- 15: Schraube
- 16: Langloch
- 17: Einführungskopf
- 18: Stützwände des Trägerelements
- 19: Nut

## Patentansprüche

1. Bandscheibenentlastungsstütze als Implantat zwischen Dornfortsätzen (7) benachbarter Wirbelkörper (6),
a) umfassend ein Trägerelement (2) mit einer Aussparung (3) und mit ersten Rückhalteflügeln (5), sowie
b) ein in die Aussparung (3) des Trägerelements (2) einschiebbares Zentralelement (4) mit zweiten Rückhalteflügeln (5'),
c) wobei im implantierten Zustand die Rückhalteflügel (5, 5') außen an den Dornfortsätzen (7) benachbarten Wirbelkörper (6) anliegen und sich die Dornfortsätze (7) auf gegenüberliegenden Druckflächen (11) auf dem Trägerelement (2) und/oder auf dem Zentralelement (4) abstützen können,
**dadurch gekennzeichnet, dass**
d) mindestens ein frei liegender Federschlitz (8), welcher transversal zur sagitalen Verlaufsrichtung der Wirbelsäule (10) ausgerichtet ist und sich über den Bereich zwischen zwei gegenüberliegenden Druckflächen (11) erstreckt, in mindestens einem der Elemente (2, 4) derart angeordnet ist, dass zwischen dem Federschlitz (8) und einer der Druckflächen (11) eine federnde Brücke (9) ausgestaltet ist,
e) durch mindestens zwei Federschlitze (8) zwei Brücken (9) ausgestaltet sind, die je einem Dornfortsatz (7) zugeordnet sind,
f) alle Federschlitze (8) parallel zueinander verlaufen, und
g) alle Federschlitze (8), die derselben Druckfläche (11) zugeordnet sind, fluchtend hintereinander liegen.

2. Bandscheibenentlastungsstütze nach Anspruch 1, **dadurch gekennzeichnet, dass** jede Druckfläche (11) jedes Elementes (2, 4) durch einen Federschlitz (8) jeweils zu einer Brücke (9) ausgestaltet ist, sodass im implantierten Zustand beide Dornfortsätze (7) auf den federnden Druckflächen (11) aufliegen.

3. Bandscheibenentlastungsstütze nach einem der vorhergehenden Ansprüche, **dadurch gekennzeichnet, dass** sich die Federschlitze (8) im Wesentlichen über den ganzen Bereich zwischen den Druckflächen (11) erstrecken.

4. Bandscheibenentlastungsstütze nach einem der vorhergehenden Ansprüche, **dadurch gekennzeichnet, dass** das Trägerelement (2) zwei über einem Einführungskopf miteinander verbundene parallele Stützwände aufweist, in denen je zwei Federschlitze (8) angebracht sind.

5. Bandscheibenentlastungsstütze nach Anspruch 4, **dadurch gekennzeichnet, dass** das Zentralelement (4) zwei Federschlitze (8) aufweist.

6. Bandscheibenentlastungsstütze nach einem der vorhergehenden Ansprüche, **dadurch gekennzeichnet, dass** alle Brücken (9) bis zu den Federschlitzen (8) dieselbe Dicke aufweisen.

## Claims

1. Intervertebral disc strain-relief support as an implant between spinous processes (7) of adjacent vertebral bodies (6),
a) comprising a carrier element (2) with an opening (3) and with first retaining wings (5), and
b) a central element (4) that has second retaining wings (5') and is insertable into the opening (3) of the carrier element (2),
c) wherein, in the implanted state, the retaining wings (5, 5') rest outside on the spinous processes (7) of adjacent vertebral bodies (6) and the spinous processes (7) is supportable on opposing pressure surfaces (11) on the carrier element (2) and/or on the central element (4),
**characterized in that**
d) at least one exposed spring slot (8), which is oriented transversely to the sagittal running direction of the spinal column (10), and extends over the region between two opposing pressure surfaces (11), is arranged in at least one of the elements (2, 4) in such a way that a resilient bridge (9) is formed between the spring slot (8) and one of the pressure surfaces (11),
e) two bridges (9), which are each allocated to a spinous process (7), are formed by at least two spring slots (8),
f) all the spring slots (8) run parallel to one another, and
g) all the spring slots (8), which are allocated to the same pressure surface, lie aligned one behind the other.

2. Intervertebral disc strain-relief support according to claim 1, **characterized in that** each pressure surface (11) of each element (2, 4) is formed by a spring slot (8) into a bridge (9), in each case, so that in the implanted state, the two spinous processes (7) rest on the resilient pressure surfaces (11).

3. Intervertebral disc strain-relief support according to any one of the preceding claims, **characterized in that** the spring slots (8) extend substantially over the entire region between the pressure surfaces (11).

4. Intervertebral disc strain-relief support according to any one of the preceding claims, **characterized in that** the carrier element (2) has two parallel support walls, which are connected to one another by means of an introduction head and in each of which two spring slots (8) are provided.

5. Intervertebral disc strain-relief support according to claim 4, **characterized in that** the central element (4) has two spring slots (8).

6. Intervertebral disc strain-relief support according to any one of the preceding claims, **characterized in that** all the bridges (9) up to the spring slots (8) have the same thickness.

## Revendications

1. Support pour soulager les disques intervertébraux, sous forme d'implant entre des corps vertébraux (6) au voisinage des apophyses épineuses (7),
a) comprenant un élément porteur (2) comportant un évidement (3) et comportant un premier épaulement de retenue (5), ainsi
b) qu'un élément central (4), pouvant être inséré dans l'évidement (3) de l'élément porteur (2), comprenant un second épaulement de retenue (5'),
c) à l'état implanté, les épaulements de retenue (5, 5') étant adjacents à l'extérieur aux corps vertébraux (6) au voisinage des apophyses épineuses (7), et les apophyses épineuses (7) pouvant s'appuyer sur des surfaces de pression (11) opposées sur l'élément porteur (2) et/ou sur l'élément central (4),
**caractérisé en ce**
d) **qu'**au moins une fente à ressort (8), à l'état libre, orientée transversalement par rapport à la direction sagittale de la colonne vertébrale (10), et qui s'étend dans un domaine entre deux surfaces de pression 11) opposées, est disposée dans au moins l'un des éléments (2, 4) de telle sorte qu'un pontage élastique (9) est créé entre la fente à ressort (8) et l'une des surfaces de pression (11),
e) **que** deux pontages (9), qui sont chacun affectés à une apophyse épineuse (7), sont créés entre au moins deux fentes à ressort (8),
f) **que** toutes les fentes à ressort (8) s'étendent parallèles les unes par rapport aux autres, et
g) **que** toutes les fentes à ressort (8) affectées à la même surface de pression (11) s'étendent alignées les unes derrière les autres.

2. Support pour soulager les disques intervertébraux selon la revendication 1, **caractérisé en ce que** chaque surface de pression (11) de chaque élément (2, 4) est conçue pour former un pontage par l'intermédiaire d'une fente à ressort (8), de sorte qu'à l'état implanté, les deux apophyses épineuses (7) s'appuient sur les surfaces de pression (11) élastiques.

3. Support pour soulager les disques intervertébraux selon l'une des revendications précédentes, **caractérisé en ce que** les fentes à ressort (8) s'étendent pratiquement sur tout le domaine entre les surfaces de pression (11).

4. Support pour soulager les disques intervertébraux selon l'une des revendications précédentes, **caractérisé en ce que** l'élément porteur (2) présente deux parois de soutien parallèles reliées entre elles par une tête d'insertion, dans lesquelles, chaque fois, deux fentes à ressort (8) sont rapportées.

5. Support pour soulager les disques intervertébraux selon la revendication 4, **caractérisé en ce que** l'élément central (4) présente deux fentes à ressort (8).

6. Support pour soulager les disques intervertébraux selon l'une des revendications précédentes, **caractérisé en ce que** tous les pontages (9) présentent la même épaisseur jusqu'aux fentes à ressort (8).
